(19) Europäisches Patentamt
European Patent Office
Office européen des brevets

(11) Veröffentlichungsnummer: **0 234 323**
**A1**

(12) **EUROPÄISCHE PATENTANMELDUNG**

(21) Anmeldenummer: **87101242.3**

(22) Anmeldetag: **29.01.87**

(51) Int. Cl.³: **C 07 D 233/40**
C 07 D 233/42, C 07 D 471/04
C 07 D 513/04, C 07 D 498/04
A 01 N 43/50, A 01 N 43/90

(30) Priorität: **08.02.86 DE 3604042**

(43) Veröffentlichungstag der Anmeldung:
**02.09.87 Patentblatt 87/36**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Liebl, Rainer, Dr.**
**An der Weinleite 5b**
**D-8901 Todtenweis(DE)**

(72) Erfinder: **Handte, Reinhard, Dr.**
**Theilweg 23**
**D-8901 Gablingen(DE)**

(72) Erfinder: **Mildenberger, Hilmar, Dr.**
**Fasanenstrasse 24**
**D-6233 Kelkheim (Taunus)(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(54) **Imidazolidin(thi)on-Derivate, Verfahren zu ihrer Herstellung sowie ihre Verwendung im Pflanzenschutz.**

(57) Die Verbindungen der Formel I

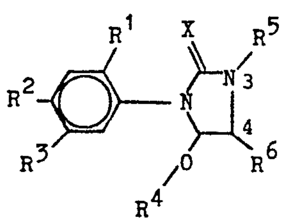

I,

worin X Sauerstoff oder Schwefel; $R^1$ Wasserstoff oder Halogen; $R^2$, $R^3$ Wasserstoff, Halogen, (Halogen)alkyl, (subst.)Alkoxy, Cycloalkoxy, (Halogen)alkenyloxy, Cycloalkenyloxy, (Halogen)alkinyloxy, Alkoxyalkoxy, (subst.) Phenoxy, (subst.) Phenylthio, (subst.) Phenylsulfinyl, (subst.) Phenylsulfonyl, (subst.) Pyridyloxy, (subst.) Benzyloxy, (subst.) Phenoxymethyl, Alkoxycarbonyl, (subst.) Alkylthio, $NO_2$ oder CN, $R^4$ Wasserstoff, (Halogen)alkyl, Alkenyl, Cycloalkyl, Cycloalkenyl, Alkinyl, Alkoxyalkyl, Alkoxycarbonylalkyl, Alkylthioalkyl, (subst.) Benzyl oder einen Rest der Formel $-C(X)-NHR^7$; $R^5$ Wasserstoff, (subst.) Alkyl, Alkenyl, Alkinyl, Cycloalkyl oder eine (subst.) Alkylenkette, die an C-Atom (4) gebunden ist und worin eine $CH_2$-Gruppe durch O oder S ersetzt sein kann, $R^6$ Wasserstoff oder Alkyl bedeuten, besitzen vorteilhafte herbizide Wirkungen. Ausgenommen von diesen Verbindungen sind solche, bei denen, falls $R^5$ für $CH_3$, X für O und $R^1$ für Wasserstoff steht, $R^2$ und $R^3$ H, Halogen, $(C_1-C_4)$Alkyl, $CF_3$, $(C_1-C_4)$Alkoxy, Phenoxy, Chlorphenoxy oder Benzyloxy bedeuten.

EP 0 234 323 A1

Imidazolidin(thi)on-Derivate, Verfahren zu ihrer Herstellung sowie ihre Verwendung im Pflanzenschutz

Herbizide mit einer Imidazolidinon-Struktur sind aus der Internationalen Patentanmeldung WO/01383 bekannt. Es wurden nun neue Imidazolin(thi)on-Derivate mit vorteilhaften Herbizideigenschaften gefunden.

Gegenstand der vorliegenden Erfindung sind daher die Verbindungen der Formel I

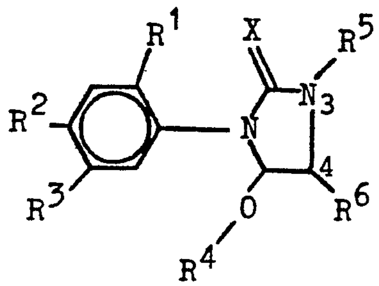

I,

worin

X Sauerstoff oder Schwefel;

$R^1$ Wasserstoff oder Halogen;

$R^2$, $R^3$ unabhängig voneinander Wasserstoff, Halogen, $(C_1-C_4)$Alkyl, $(C_1-C_4)$Halogenalkyl, $(C_1-C_4)$-Alkoxy, $(C_1-C_4)$Halogenalkoxy, $(C_3-C_6)$Cycloalkoxy, $(C_2-C_4)$-Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$-Alkoxy-$(C_1-C_4)$alkoxy; $(C_1-C_4)$Alkoxycarbonyl$(C_1-C_4)$alkoxycarbonyl, $(C_1-C_4)$Halogenalkoxycarbonyl, $(C_1-C_4)$Alkylsulfinyl, $(C_1-C_4)$Alkylsulfonyl; Phenoxy, Phenylthio, Phenylsulfinyl, Phenylsulfonyl, wobei der Phenylring jeweils ein- bis dreifach durch Halogen, $(C_1-C_4)$Alkyl, $CF_3$ oder $NO_2$ substituiert sein kann; Pyridyloxy, das ein- oder zweifach durch Halogen oder $CF_3$ substituiert sein kann; Benzyloxy, Phenoxymethyl, die beide im Phenylring ein- bis dreifach durch Halogen, $(C_1-C_4)$Alkyl, $(C_1-C_4)$Alkoxy, $CF_3$ oder $NO_2$ substituiert sein können; $(C_1-C_4)$Alkoxycarbonyl, $(C_1-C_4)$Alkoxycarbonyl$(C_1-C_4)$-alkoxy, $(C_1-C_4)$Alkylthio, $(C_1-C_4)$Alkoxycarbonyl$(C_1-C_4)$-alkylthio, $NO_2$ oder CN,

$R^4$ Wasserstoff, $(C_1-C_4)$Alkyl, Halogen$(C_1-C_4)$alkyl, $(C_3-C_4)$Alkenyl, $(C_3-C_4)$Cycloalkyl, $(C_5-C_6)$Cycloalkenyl, $(C_3-C_4)$Alkinyl, $(C_1-C_4)$Alkoxy-$(C_1-C_4)$alkyl, $(C_1-C_4)$Alkoxycarbonyl$(C_1-C_4)$alkyl, $(C_1-C_4)$Alkylthio$(C_1-C_4)$alkyl; Benzyl, das ein- bis dreifach durch Halogen, $(C_1-C_4)$-Alkyl, $(C_1-C_4)$Alkoxy, $(C_1-C_4)$Alkoxycarbonyl, $CF_3$, $NO_2$ oder CN substituiert sein kann oder einen Rest der Formel $-\underset{\underset{X}{\|}}{C}-NH-R^7$ ;

$R^5$ Wasserstoff, $(C_1-C_4)$Alkyl, $(C_3-C_4)$Alkenyl, $(C_3-C_4)$-Alkinyl, $(C_3-C_6)$Cycloalkyl, $(C_1-C_4)$Alkoxy$(C_1-C_4)$-alkyl, $(C_1-C_4)$Alkylthio$(C_1-C_4)$alkyl, $(C_1-C_4)$Alkoxycarbonyl$(C_1-C_4)$alkyl oder eine $(C_4-C_5)$Alkylenkette, die das N-Atom (3) mit dem C-Atom (4) des Imidazolin(thi)on-Ringes verknüpft und bei der eine $CH_2$-Gruppe durch Sauerstoff oder Schwefel substituiert sein kann und die bis zu zweifach durch $(C_1-C_4)$Alkyl, $(C_1-C_4)$Alkoxycarbonyl, $(C_1-C_4)$Alkoxy$(C_1-C_4)$alkoxycarbonyl oder $(C_1-C_4)$Alkoxycarbonyl$(C_1-C_4)$alkoxycarbonyl substituiert sein kann;

$R^6$ Wasserstoff oder $(C_1-C_4)$Alkyl,

$R^7$ $(C_1-C_8)$Alkyl; Phenyl oder Benzyl, jeweils bis zu dreifach durch Halogen, $(C_1-C_4)$Alkyl, $(C_1-C_4)$Alkoxy, $CF_3$, $NO_2$ oder $(C_1-C_4)$Alkoxycarbonyl substituiert sein können, bedeuten, mit der Maßgabe, daß wenn $R^5$ für $CH_3$, X für Sauerstoff und $R^1$ für Wasserstoff steht, $R^2$ und $R^3$ nicht H, Halogen, $(C_1-C_4)$Alkyl, $CF_3$, $(C_1-C_4)$Alkoxy, Phenoxy, Chlorphenoxy, Benzyloxy bedeuten dürfen.

Bevorzugte Verbindungen der Formel I sind solche, bei denen

$R^2$ Fluor, Chlor, Brom, $(C_1-C_4)$Alkoxy, $(C_1-C_4)$Halogenalkoxy, $(C_1-C_4)$Alkylthio, $(C_1-C_4)$Alkylsulfinyl, $(C_1-C_4)$Alkylsulfonyl oder $NO_2$;

$R^3$ Wasserstoff, Chlor, Brom, $(C_1-C_4)$Alkyl, $(C_1-C_4)$Halogenalkyl, $(C_1-C_4)$Alkoxy, $(C_1-C_4)$Halogenalkoxy, $(C_3-C_4)$-Alkinyloxy, $(C_1-C_4)$Alkylthio, $(C_1-C_4)$Alkoxycarbonyl-$(C_1-C_4)$alkylthio oder $(C_1-C_4)$Alkoxycarbonyl$(C_1-C_4)$-alkoxy;

$R^4$ Wasserstoff, $(C_1-C_4)$Alkyl, Benzyl, das ein- bis dreifach durch Halogen oder $(C_1-C_4)$Alkyl substituiert ist, oder $-\underset{\overset{\|}{O}}{C}-NHR^7$,

$R^5$ $(C_1-C_4)$Alkyl oder Butylen, wobei eine $CH_2$-Gruppe durch Sauerstoff oder Schwefel ersetzt sein kann und das bis zu zweifach durch $(C_1-C_4)$Alkyl substituiert sein kann,

$R^6$ = H und

$R^7$ $(C_1-C_4)$Alkyl

bedeuten.

Falls $R^5$ eine $(C_4-C_5)$Alkylenkette bedeutet, bei der eine $CH_2$-Gruppe durch C oder S substituiert ist, kommt insbesondere in Betracht der Rest $-CH_2-CH_2-O-CH_2-$ oder $-CH_2-CH_2-S-CH_2-$, der durch $(C_1-C_4)$Alkyl substituiert sein kann.

Als besonders bevorzugte Verbindungen der Formel I sind solche anzusehen, bei denen $R^1$ = F, $R^2$ = F, Cl, Br, $(C_1-C_4)$Alkoxy, $(C_1-C_4)$Halogenalkoxy, $(C_1-C_4)$Alkylthio oder $(C_1-C_4)$Alkylsulfonyl; $R^3$ = $(C_1-C_4)$Alkoxy, $(C_1-C_4)$Halogenalkoxy, $(C_1-C_4)$Alkylthio, $(C_1-C_4)$Alkoxycarbonyl$(C_1-C_4)$-alkoxy, $(C_1-C_4)$Alkoxycarbonyl$(C_1-C_4)$alkylthio oder Propargyloxy, $R^4$ = H, $R^5$ = $(C_1-C_4)$Alkyl, $-CH_2-CH_2-CH_2-CH_2-$ , $-CH_2-CH_2-O-\underset{(\alpha)}{C}H_2-$ oder $-CH_2-CH_2-S-\overset{(\alpha)}{C}H_2-$ , wobei die letzten drei Reste durch $(C_1-C_4)$Alkyl ein- bis zweifach substituiert sein kann und das C-Atom α dieser Reste an C-Atom (4) des Imidazolin(thi)on-Ringes geknüpft ist, und $R^6$ = H bedeuten.

Die Erfindung umfaßt auch alle Stereoisomeren oder deren Gemische der Verbindungen der Formel I.

Halogenalkyl, -alkenyl, -alkinyl, -alkoxy bedeutet, daß die betreffenden aliphatischen Reste durch ein- oder mehrere Chlor-, Fluor- oder Bromatome substituiert sind. Hierunter zählen beispielsweise $CF_3$, 1,1,2,2-Tetrafluorethoxy-, 2,2-Dichlor-1,1-difluor-ethoxy oder 2-Chlor-1,1,2-trifluor-ethoxy, $-O-C(Cl){=}CHCl$, $-OCF_2-CFBrH$, $-O-CH_2-CH_2-Cl$, $-OCF_2-CFH-CF_3$, $-CF_2Cl$, $-CF_2CHF_2$, $-CH_2CF_3$, $-CF_2CHFCl$, $-CF_2CHF-CF_3$.

Ein weiterer Gegenstand der Anmeldung sind Verfahren zur Herstellung der Verbindungen der Formel I, dadurch gekennzeichnet, daß man eine Verbindung der Formel II reduziert und die so erhaltenen Verbindungen I mit $R^4$ = Wasserstoff gewünschtenfalls am Sauerstoff zu den übrigen Verbindungen der Formel I verestert.

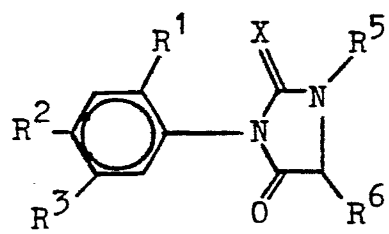

II

Als Reduktionsmittel für diese Umsetzung dienen vorzugsweise Metallhydride wie z.B. Lithiumaluminiumhydrid oder Natriumborhydrid. Die Reduktion wird bei Verwendung von Natriumborhydrid in einem niederen Alkohol, einem Alkohol-Wasser-Gemisch oder einem Ether, wie z.B. Tetrahydrofuran, Diethylether, Dimethoxyethan als Lösungsmittel durchgeführt. Bei Verwendung von Lithiumaluminiumhydrid wird die Reaktion in wasserfreiem Ether wie THF, Dimethoxyethan durchgeführt.

Die Temperaturen für die Reduktion liegen bei 0 - 80°C, vorzugsweise bei 10 - 40°C.

Die Veresterung der Verbindungen I mit $R^4$ = OH kann mit einem entsprechenden Alkylierungsmittel in Gegenwart einer

anorganischen Base wie z.B. Kaliumcarbonat, Alkalimetallhydroxid oder auch Natriumhydrid oder in Gegenwart einer organischen Base wie z.B. Triethylamin oder Pyridin erfolgen. Die Reaktionstemperaturen können zwischen Raumtemperatur und dem Siedepunkt des Lösungsmittels variieren.
Als Lösungsmittel hierfür eignen sich alle unter den Reaktionsbedingungen inerten organischen Lösungsmittel. Als Beispiele seien genannt: Toluol, Acetonitril, Dimethylformamid, Aceton.

Ferner können die Verbindungen I (R = OH) mit einem Iso(thio)cyanat in einem unter den Reaktionsbedingungen inerten organischen Lösungsmittel wie z.B. Toluol, Aceton, Acetonitril, vorzugsweise in Gegenwart katalytischer Mengen einer organischen Base, wie z.B. Triethylamin, Pyridin oder auch 1,4-Diaza-bicyclo[2.2.2]octan carbamoyliert werden.

Die Ausgangsprodukte der Formel II sind zum Teil bekannt (EP-A 70 389, EP-A 104 532) oder lassen sich auf einfache, dem Fachmann geläufige Art aus den entsprechenden Iso(thio)cyanaten und Piperidin-2-carbonsäuren oder deren Ester bzw. N-substituierten Glycinen herstellen, s. hierzu, Beiträge zur chemischen Physiologie und Pathologie 11, S. 160.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

...

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol

...

oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprüh-

...

bare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachfolgende Beispiele dienen zur Erläuterung der Erfindung.

A. Formulierungsbeispiele

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

B. Chemische Beispiele

Beispiel 1

3-(4-Chlor-2-fluor-5-methoxy-phenyl)-4-hydroxy-1-methyl-imidazolidin-2-on

Man löst 27,3 g (0,10 mol) 3-(4-Chlor-2-fluor-5-methoxy-phenyl)-1-methyl-imidazolidin-2,4-dion in 200 ml Methanol und gibt 5,7 g (0,15 mol) Natriumborhydrid portionsweise zu, so daß die Temperatur auf 30 - 35 °C steigt. Nach beendeter Zugabe wird 1 h bei 30 °C nachgerührt. Man gießt auf 500 ml Eiswasser und saugt den ausgefallenen Niederschlag ab. Nach dem Trocknen erhält man 25,2 g (92 % d. Th.) 3-(4-Chlor-2-fluor-5-methoxy-phenyl)-4-hydroxy-1-methyl-imidazolidin-2-on in Form farbloser Kristalle mit Schmp. 138 - 140 °C.

./11

Beispiel 2

4-Hydroxy-1-methyl-3-[3-methyl-4-(1,1,2,2-tetrafluorethoxy)-phenyl]-imidazolidin-2-on

Man löst 16,0 g (0,05 mol) 1-Methyl-3-[3-methyl-4-(1,1,2,2-tetrafluorethoxy)-phenyl]-imidazolidin-2,4-dion in 100 ml Isopropanol und gibt 2,85 g (0,075 mol) Natriumborhydrid so zu, daß die Temperatur auf 40 °C steigt. Nach beendeter Zugabe wird noch 30 min bei 40 °C nachgerührt und auf 300 ml Eiswasser gegossen. Man extrahiert 2mal mit je 100 ml Methylenchlorid, wäscht die vereinigten Methylenchloridphasen mit 100 ml Wasser und trocknet über Natriumsulfat. Nach Abdestillieren des Lösungsmittels erhält man 15,4 g (96 % d. Th.) 4-Hydroxy-1-methyl-3-[3-methyl-4-(1,1,2,2-tetrafluorethoxy)-phenyl]-imidazolidin-2-on in Form eines hellgelben zähen Öls.

Beispiel 3

1-Butyl-3-(4-chlor-2-fluor-5-methoxy-phenyl)-4-hydroxy-imidazolidin-2-thion

Man löst 33,3 g (0,10 mol) 1-Butyl-3-(4-chlor-2-fluor-5-methoxy-phenyl)-4-oxo-2-thioxo-imidazolidin in 300 ml Methanol und gibt 5,7 g (0,15 mol) Natriumborhydrid so zu, daß die Temperatur auf 30 °C ansteigt. Nach beendeter Zugabe wird noch 30 min bei 30 °C gerührt und auf 800 ml Eiswasser gegossen. Der Niederschlag wird abgesaugt und getrocknet. Man erhält 31,8 g (95 % d. Th.) 1-Butyl-3-(4-chlor-2-fluor-5-methoxy-phenyl)-4-hydroxy-imidazolidin-2-thion in Form farbloser Kristalle mit Schmp. 137 - 138 °C.

Beispiel 4

3-(4-Chlor-2-fluor-5-methoxy-phenyl)-1-methyl-4-(N-methyl-carbamoyloxy)-imidazolidin-2-on

Man löst 27,5 g (0,10 mol) 3-(4-Chlor-2-fluor-5-methoxy-phenyl)-4-hydroxy-1-methyl-imidazolidin-2-on (Bsp. 1) in 200 ml Acetonitril, gibt 50 mg Diazabicyclo[2.2.2]octan dazu und tropft bei 20 °C 6,84 g (0,12 mol) Methylisocyanat zu. Man rührt 5 h bei 50 °C und destilliert das Lösungsmittel ab. Der verbleibende feste Rückstand wird in 200 ml Ether aufgekocht und im Eisbad abgekühlt. Der Niederschlag wird abgesaugt und getrocknet. Man erhält 31,0 g (93 % d. Th.) 3-(4-Chlor-2-fluor-5-methoxy-phenyl)-1-methyl-4-(N-methyl-carbamoyloxy)-imidazolidin-2-on in Form farbloser Kristalle mit Schmp. 128 - 131 °C.

Beispiel 5

4-Benzyloxy-1-methyl-3-[3-methyl-4-(1,1,2,2-tetrafluorethoxy)-phenyl]-imidazolidin-2-on

Man löst 16,1 g (0,05 mol) 4-Hydroxy-1-methyl-3-[3-methyl-4-(1,1,2,2-tetrafluorethoxy)-phenyl]-imidazolidin-2-on in 150 ml Acetonitril und gibt bei 5 °C 1,50 g Natriumhydrid (80%ig in Weißöl) zu. Man rührt 10 min bei 5 °C und tropft anschließend bei 5 °C 6,40 g (0,05 mol) Benzylchlorid zu. Man rührt 5 h bei 50 °C und gießt auf 400 ml Eiswasser. Man extrahiert 2mal mit je 100 ml Methylenchlorid, wäscht die vereinigten Methylenchloridphasen mit 100 ml Wasser und trocknet über Natriumsulfat. Nach Abdestillieren des Lösungsmittels erhält man 19,4 g (94 % d. Th.) 4-Benzyloxy-1-methyl-3-[3-methyl-4-(1,1,2,2-tetrafluorethoxy)-phenyl]-imidazolidin-2-on in Form eines hellgelben Öles.

Beispiel 6

8-(4-Chlor-2-fluor-5-methoxy-phenyl)-7-hydroxy-9-oxo-1,8-diaza-bicyclo[4.3.0]nonan

31,3 g (0,10 mol) 8-(4-Chlor-2-fluor-5-methoxy-phenyl)-7,9-dioxo-1,8-diaza-bicyclo[4.3.0]nonan wurden in 200 ml Methanol gelöst. Man gab portionsweise 5,7 g (0,15 mol) Natriumborhydrid so zu, daß die Temperatur bei 30 - 40 °C gehalten wurde. Nach beendeter Zugabe wurde noch 1 h bei 30 °C gerührt und auf 500 ml Wasser gegossen. Der ausgefallene Niederschlag wurde abgesaugt und getrocknet. Man erhielt 29,8 g (94 % d. Th.) 8-(4-Chlor-2-fluor-5-methoxy-phenyl)-7-hydroxy-9-oxo-1,8-diaza-bicyclo[4.3.0]nonan in Form farbloser Kristalle mit Schmp. 155 - 157°C.

Beispiel 7

8-(4-Chlor-2-fluor-5-methoxy-phenyl)-7-hydroxy-9-thioxo-1,8-diaza-bicyclo[4.3.0]nonan

32,9 g (0,10 mol) 8-(4-Chlor-2-fluor-5-methoxy-phenyl)-7-oxo-9-thioxo-1,8-diaza-bicyclo[4.3.0]nonan wurden in 250 ml Ethanol gelöst. Man gab portionsweise 5,7 g (0,15 mol) Natriumborhydrid so zu, daß die Temperatur bei 30 °C gehalten wurde. Nach beendeter Zugabe wurde noch 30 min bei 30 °C nachgerührt und auf 600 ml Eiswasser gegossen. Der Niederschlag wurde abgesaugt, getrocknet und aus Methanol umkristallisiert. Man erhielt 24,9 g (75 % d. Th.) 8-(4-Chlor-2-fluor-5-methoxy-phenyl)-7-hydroxy-9-thioxo-1,8-diaza-bicyclo[4.3.0]nonan in Form farbloser Kristalle mit Schmp. 208 - 215 °C.

In analoger Weise lassen sich die Verbindungen der nachfolgenden Tabellen IA, IB und IC erhalten.

Tabelle 1A

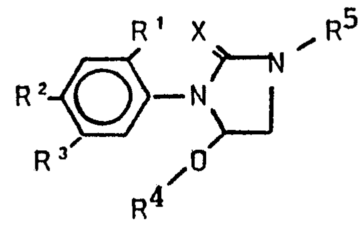

| Bsp. | $R^1$ | $R^2$ | $R^3$ | $R^5$ | $R^4$ | X | Fp [°C] |
|---|---|---|---|---|---|---|---|
| 8 | F | Cl | $OC_2H_5$ | $n-C_4H_9$ | $CH_3$ | O | |
| 9 | F | Cl | $-OCH_3$ | $-i-C_3H_7$ | H | O | gelbes Oel |
| 10 | F | Cl | " | $CH_3$ | $CH_3$ | O | " |
| 11 | F | Cl | " | " | $-CH_2-C_6H_5$ | O | " |
| 12 | F | Cl | $-O-CH_2-CO_2C_2H_5$ | " | H | O | |
| 13 | F | Cl | $-OCH_2CH_2OCH_3$ | " | H | O | |
| 14 | F | Cl | $-SCH_3$ | " | H | O | |
| 15 | F | Cl | $-S-CH_2-CO_2C_2H_5$ | " | H | O | |
| 16 | F | Cl | $-O-i-C_3H_7$ | " | $-C(=O)-NHCH_3$ | O | |
| 17 | F | Cl | $-OCH_3$ | " | " | S | |
| 18 | F | Cl | $-O-i-C_3H_7$ | " | $-C(=O)-NHC_2H_5$ | S | |
| 19 | F | Cl | $-OCH_3$ | $-i-C_3H_7$ | H | S | |
| 20 | F | Cl | $-O-CH_2-CO_2C_2H_5$ | $CH_3$ | H | S | |

0234323

Fortsetzung Tabelle 1A

| Bsp. | $R^1$ | $R^2$ | $R^3$ | $R^5$ | $R^4$ | X | Fp [°C] |
|---|---|---|---|---|---|---|---|
| 21 | F | Cl | $-O-CH_2CH_2-OCH_3$ | $CH_3$ | H | S | |
| 22 | F | Cl | $-O-CH_2-O-CH_3$ | " | H | S | |
| 23 | F | Cl | $-OCH_3$ | " | $CH_3$ | S | |
| 24 | F | Cl | " | " | $-CH_2-C_6H_5$ | S | |
| 25 | Cl | Cl | $-CO_2C_2H_5$ | " | H | O | gelbes Harz |
| 26 | Cl | Cl | " | " | H | S | |
| 27 | H | $-OC_2H_5$ | $-CO_2C_2H_5$ | $CH_3$ | H | O | |
| 28 | H | " | $-CF_3$ | $-i-C_3H_7$ | H | O | |
| 29 | H | $-OCH_3$ | " | $CH_3$ | H | S | |
| 30 | H | $-OCH_2OCH_3$ | " | " | H | O | |
| 31 | H | $-OC_2H_5$ | $-CN$ | " | H | O | |
| 32 | H | " | $-NO_2$ | " | H | O | |
| 33 | H | " | $-CF_3$ | " | H | S | |
| 34 | H | " | " | $-i-C_3H_7$ | H | S | |
| 35 | H | " | " | $CH_3$ | $-C(=O)-NHCH_3$ | S | |
| 36 | H | " | " | " | " | S | |
| 37 | H | " | " | " | $CH_3$ | S | |

Fortsetzung Tabelle 1A

| Bsp. | $R^1$ | $R^2$ | $R^3$ | $R^5$ | $R^4$ | X | Fp [°C] |
|---|---|---|---|---|---|---|---|
| 38 | H | $-OC_2H_5$ | $-CF_3$ | $CH_3$ | $-CH_2-C_6H_5$ | S | |
| 39 | H | " | " | " | $-i-C_3H_7$ | S | |
| 40 | H | $-O-CF_2-CHCl_2$ | $CH_3$ | $CH_3$ | H | O | 125-126 |
| 41 | H | " | " | " | $CH_3$ | O | |
| 42 | H | " | " | " | $-CH_2-C_6H_5$ | O | |
| 43 | H | " | " | " | $-C(=O)-NHCH_3$ | O | |
| 44 | H | $-OF_2-CHF_2$ | " | " | H | O | gelbes Harz |
| 45 | H | " | " | " | $-CH_2-C_6H_5$ | O | " " |
| 46 | H | " | " | " | $CH_3$ | O | " " |
| 47 | H | " | " | " | $-C(=O)-NHCH_3$ | O | " " |
| 48 | H | $-O-CF_2-CHFCl$ | " | " | H | O | |
| 49 | H | $-OCF_3$ | " | " | H | O | |
| 50 | H | $-O-CCl=CHCl$ | " | " | H | O | |
| 51 | H | $-O-CF_2-CFBrH$ | " | " | H | O | |
| 52 | H | $-O-CH_2-CH_2Cl$ | " | " | H | O | |
| 53 | H | $-O-CF_2-CFH-CF_3$ | " | " | H | O | |

Tabelle 1B

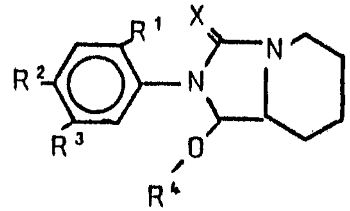

| Bsp. | $R^1$ | $R^2$ | $R^3$ | $R^4$ | X | Fp [°C] |
|---|---|---|---|---|---|---|
| 54 | F | Cl | $-O-i-C_3H_7$ | H | O | |
| 55 | F | Cl | $-O-CH_2-CO_2C_2H_5$ | H | O | Oel |
| 56 | F | Cl | $-O-CH(CH)_3-CO_2C_2H_5$ | H | O | " |
| 57 | F | Cl | $-S-CH_2-CO_2C_2H_5$ | H | O | |
| 58 | F | Cl | $-S-C_2H_5$ | H | O | |
| 59 | F | Cl | $-OCH_3$ | $-\underset{\underset{O}{\|}}{C}-NHCH_3$ | O | |
| 60 | F | Cl | " | $-CH_2-C_6H_5$ | O | |
| 61 | F | Cl | " | $-CH_3$ | O | |
| 62 | H | Br | H | H | O | 152-154 |
| 63 | H | Br | H | $-\underset{\underset{O}{\|}}{C}-NHCH_3$ | O | |
| 64 | H | Br | H | $CH_3$ | O | |

Fortsetzung Tabelle 1B

| Bsp. | $R^1$ | $R^2$ | $R^3$ | $R^4$ | X | Fp [°C] |
|---|---|---|---|---|---|---|
| 65 | H | $-O-CF_2-CHF_2$ | H | H | O | 178-180 |
| 66 | H | $-O-CF_2-CHF_2$ | $CH_3$ | H | O | |
| 67 | H | $-O-CF_2-CF_2-CF_3$ | " | H | O | |
| 68 | H | $-O-CF_2-CHFCl$ | " | H | O | |
| 69 | H | $-O-CF_2-CHCl_2$ | " | H | O | |
| 70 | H | $-OC_2H_5$ | $CF_3$ | H | O | |
| 71 | H | $-OC_2H_5$ | " | $-C(=O)-NHCH_3$ | O | |
| 72 | H | " | $-CO_2CH_3$ | H | O | |
| 73 | H | " | CN | H | O | |
| 74 | H | " | $NO_2$ | H | O | |
| 75 | H | Cl | Cl | H | O | |
| 76 | H | $CH_3$ | Cl | H | O | 169-172 |
| 77 | F | F | H | H | O | |
| 78 | F | F | $CO_2C_2H_5$ | H | O | |
| 79 | F | Cl | $-CO_2C_2H_5$ | H | O | |
| 80 | Cl | Cl | $-CO_2C_2H_5$ | H | O | 158-160 |
| 81 | H | Cl | $-OCH_3$ | H | O | |

Fortsetzung Tabelle 1B

| Bsp. | $R^1$ | $R^2$ | $R^3$ | $R^4$ | X | Fp [°C] |
|---|---|---|---|---|---|---|
| 82 | H | Br | $-OCH_3$ | H | O | |
| 83 | H | Cl | $-CO_2C_2H_5$ | H | O | Sirup |
| 84 | H | Br | " | H | O | |
| 85 | H | Cl | " | $-C(=O)-NHCH_3$ | O | |
| 86 | F | Cl | $-O-iC_3H_7$ | H | S | |
| 87 | F | Cl | $-OCH_3$ | $-C(=O)-NHCH_3$ | S | |
| 88 | F | Cl | " | $-CH_3$ | S | |
| 89 | F | Cl | " | $-CH_2-C_6H_5$ | S | |
| 90 | H | Cl | $-CO_2C_2H_5$ | H | S | |
| 91 | H | Br | " | H | S | |
| 92 | H | Br | $-OCH_3$ | H | S | |
| 93 | H | Cl | " | H | S | |
| 94 | H | Cl | H | $CH_3$ | O | |
| 95 | H | Cl | H | H | O | 138-143 |
| 96 | H | Cl | H | $-C(=O)-NHCH_3$ | O | 158-160 |

| Bsp. | $R^1$ | $R^2$ | $R^3$ | $R^4$ | X | Fp (°C) |
|---|---|---|---|---|---|---|
| 97 | H | Cl | $-CO_2CH(CH_3)CO_2C_2H_5$ | H | O | Harz |
| 98 | H | $-OCH_2CH_2-OCH_3$ | $-CF_3$ | H | O | 135-137 |
| 99 | F | Br | $-OCH_2C\equiv CH$ | H | O | Glas |
| 100 | H | Br | $-CO_2CH(CH_3)CO_2C_2H_5$ | H | O | Glas |
| 101 | H | F | $-CO_2CH(CH_3)CO_2C_2H_5$ | H | O | Sirup |
| 102 | F | Cl | $-OCH_2-C\equiv CH$ | H | S | 198-204 |

Tabelle 1C

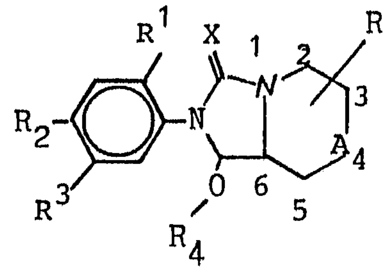

| Bsp. | $R_1$ | $R_2$ | $R_3$ | $R_4$ | X | R | A | Fp (°C) |
|---|---|---|---|---|---|---|---|---|
| 103 | F | Cl | $-OCH_3$ | H | O | $2-CH_3$ | $CH_2$ | Glas |
| 104 | F | Cl | $-OCH_2-C\equiv CH$ | H | O | $2-CH_3$ | $CH_2$ | Glas |
| 105 | F | Cl | $-CO_2CH(CH_3)CO_2C_2H_5$ | H | O | $2-CH_3$ | $CH_2$ | Glas |
| 106 | F | Cl | $-O-CH(CH_3)CO_2C_2H_5$ | H | O | $2-CH_3$ | $CH_2$ | Glas |
| 107 | H | Cl | $-CO_2C_2H_5$ | H | O | $2-CO_2CH(CH_3)CO_2C_2H_5$ | $CH_2$ | Glas |
| 108 | F | Cl | $-OCH_3$ | H | O | $3-CO_2C_2H_5$ | $CH_2$ | Glas |
| 109 | F | Cl | $-OCH_3$ | H | O | $5-C_2H_5$ | $CH_2$ | Sirup |
| 110 | F | Cl | $-OCH_3$ | H | O | $6-CH_3$ | $CH_2$ | Harz |
| 111 | H | Cl | H | H | O | $2-CH_3$ | S | Harz |
| 112 | F | Cl | $-OCH_3$ | H | O | H | S | 139-144 |
| 113 | F | Cl | $-OCH(CH_3)CO_2C_2H_5$ | H | O | H | S | Glas |
| 114 | F | Cl | $-OCH_3$ | H | S | H | S | 184-188 |
| 115 | F | Cl | $-OCH_3$ | H | O | H | O | Glas |
| 116 | F | Cl | $-OCH_3$ | H | S | H | O | Harz |

Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist.
Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

1. Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60 - 80 %).

Die optische Bonitur der Pflanzen- bzw. Auflaufschäden erfolgte nach dem Auflaufen der Versuchspflanzen nach einer Versuchszeit von 3 - 4 Wochen im Vergleich zu unbehandelten Kontrollen.

Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

2. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1 °C, relative Luftfeuchte 60 - 80 %) die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf. (Tabelle 3).

0234323

Tabelle 2 Vorauflaufwirkung der Verbindungen

| Bsp. | Dosis kg a. i./ha) | herbizide Wirkung SIA | CRS | ECG | LOM |
|---|---|---|---|---|---|
| 1 | 2,5 | 5 | 5 | 5 | 5 |
| 5 | 2,5 | 4 | 5 | 5 | 4 |
| 7 | 2,5 | 5 | 5 | 5 | 5 |
| 8 | 2,5 | 4 | 4 | 2 | 3 |
| 3 | 2,5 | 5 | 3 | 3 | 4 |
| 6 | 2,5 | 5 | 5 | 5 | 5 |
| 2 | 2,5 | 5 | 5 | 5 | 5 |
| 4 | 2,5 | 4 | 5 | 5 | 4 |
| 9 | 2,5 | 5 | 5 | 5 | 5 |
| 10 | 2,5 | 5 | 5 | 2 | 3 |
| 99 | 2,5 | 5 | 5 | 5 | 5 |
| 112 | 2,5 | 4 | 4 | 3 | 4 |

Tabelle 3 Nachauflaufwirkung der Verbindungen

| Bsp. | Dosis (kg a. i./ha) | herbizide Wirkung SIA | CRS | ECG | LOM |
|---|---|---|---|---|---|
| 7 | 2,5 | 4 | 3 | 5 | 3 |
| 3 | 2,5 | 5 | 5 | 4 | 2 |
| 6 | 2,5 | 5 | 5 | 5 | 4 |
| 2 | 2,5 | 5 | 5 | 5 | 4 |
| 5 | 2,5 | 5 | 1 | 5 | 2 |
| 9 | 2,5 | 5 | 5 | 4 | 4 |
| 55 | 2,5 | 4 | 3 | 2 | 2 |
| 99 | 2,5 | 5 | 5 | 5 | 5 |
| 112 | 2,5 | 5 | 3 | 3 | 4 |

Abkürzungen

SIA = Sinapis alba
CRS = Chrysanthemum segetum
ECG = Echinochloa crus-galli
LOM = Lolium multiflorum
a. i. = Aktivsubstanz

3. Kulturpflanzenverträglichkeit

In weiteren Versuchen im Gewächshaus wurden Samen einer größeren Anzahl von Kulturpflanzen in Töpfen in sandigem Lehmboden ausgelegt und mit Erde abgedeckt.

Ein Teil der Töpfe wurde sofort wie unter 1. beschrieben behandelt; die übrigen wurden im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z. B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

4. Herbizide Wirkung bei Anwendung in Reis

Knollen und Rhizome bzw. Jungpflanzen oder Samen von Reis und verschiedenen Reisunkräutern wie Cyperus-Arten, Eleocharis, Scirpus und Echinochloa wurden in geschlossenen Töpfen von 13 cm Durchmesser in spezielle Reiserde ausgelegt bzw. -gepflanzt und mit Wasser bis zu einer Höhe von 1 cm über dem Boden angestaut. Ebenso wurde mit Reispflanzen verfahren.

Im Vorauflaufverfahren, d. h. 3 - 4 Tage nach dem Anpflanzen wurden die erfindungsgemäßen Verbindungen in Form wäßriger Suspensionen oder Emulsionen ins Bewässerungswasser gegossen oder als Granulate ins Wasser gestreut.

./25a

0234323

Im Nachauflaufverfahren erfolgte die geschilderte Behandlung im Dreiblattstadium der Unkräuter und des Reises.

Jeweils drei Wochen später wurde die herbizide Wirkung und eine eventuelle Schadwirkung gegenüber Reis bonitiert. Die Ergebnisse zeigen, daß sich die erfindungsgemäßen Verbindungen zur selektiven Unkrautbekämpfung in Reis eignen.

Gegenüber bisherigen Reisherbiziden zeichnen sich die erfindungsgemäßen Verbindungen dadurch aus, daß sie zahlreiche, insbesondere auch schwer bekämpfbare Unkräuter, die aus Dauerorganen keimen, wirkungsvoll bekämpfen und dabei von Reis toleriert werden.

Patentansprüche:

1. Verbindungen der allgemeinen Formel I

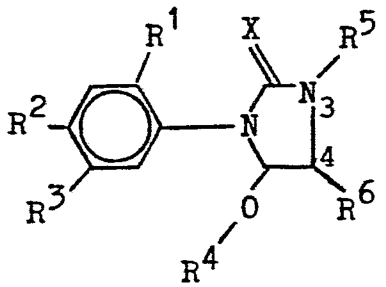

I,

worin

X Sauerstoff oder Schwefel;

$R^1$ Wasserstoff oder Halogen;

$R^2$, $R^3$ unabhängig voneinander Wasserstoff, Halogen, $(C_1-C_4)$Alkyl, $(C_1-C_4)$Halogenalkyl, $(C_1-C_4)$-Alkoxy, $(C_1-C_4)$Halogenalkoxy, $(C_3-C_6)$Cycloalkoxy, $(C_2-C_4)$-Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$-Alkoxy-$(C_1-C_4)$alkoxy; $(C_1-C_4)$Alkoxycarbonyl$(C_1-C_4)$alkoxycarbonyl, $(C_1-C_4)$Halogenalkoxycarbonyl, $(C_1-C_4)$Alkylsulfinyl, $(C_1-C_4)$Alkylsulfonyl, Phenoxy, Phenylthio, Phenylsulfinyl, Phenylsulfonyl wobei der Phenylring jeweils ein- bis dreifach durch Halogen, $(C_1-C_4)$Alkyl, $CF_3$ oder $NO_2$ substituiert sein kann; Pyridyloxy, das ein- oder zweifach durch Halogen oder $CF_3$ substituiert sein kann; Benzyloxy, Phenoxymethyl, die beide im Phenylring ein- bis dreifach durch Halogen, $(C_1-C_4)$Alkyl, $(C_1-C_4)$Alkoxy, $CF_3$ oder $NO_2$ substituiert sein können; $(C_1-C_4)$Alkoxycarbonyl, $(C_1-C_4)$Alkoxycarbonyl$(C_1-C_4)$-alkoxy, $(C_1-C_4)$Alkylthio, $(C_1-C_4)$Alkoxycarbonyl$(C_1-C_4)$-alkylthio, $NO_2$ oder CN,

$R^4$ Wasserstoff, $(C_1-C_4)$Alkyl, Halogen$(C_1-C_4)$alkyl, $(C_3-C_4)$Alkenyl, $(C_3-C_4)$Cycloalkyl, $(C_5-C_6)$Cycloalkenyl, $(C_3-C_4)$Alkinyl, $(C_1-C_4)$Alkoxy-$(C_1-C_4)$alkyl, $(C_1-C_4)$Alkoxycarbonyl$(C_1-C_4)$alkyl, $(C_1-C_4)$Alkylthio$(C_1-C_4)$alkyl; Benzyl, das ein- bis dreifach durch Halogen, $(C_1-C_4)$-Alkyl, $(C_1-C_4)$Alkoxy, $(C_1-C_4)$Alkoxycarbonyl, $CF_3$, $NO_2$ oder CN substituiert sein kann oder einen Rest der Formel $-\underset{\underset{X}{\|}}{C}-NH-R^7$ ;

$R^5$ Wasserstoff, $(C_1-C_4)$Alkyl, $(C_3-C_4)$Alkenyl, $(C_3-C_4)$-Alkinyl, $(C_3-C_6)$Cycloalkyl, $(C_1-C_4)$Alkoxy$(C_1-C_4)$-alkyl, $(C_1-C_4)$Alkylthio$(C_1-C_4)$alkyl, $(C_1-C_4)$Alkoxy-carbonyl$(C_1-C_4)$alkyl oder eine $(C_4-C_5)$Alkylenkette, die das N-Atom (3) mit dem C-Atom (4) des Imidazolin(thi)on-Ringes verknüpft und bei der eine $CH_2$-Gruppe durch Sauerstoff oder Schwefel substituiert sein kann und die bis zu zweifach durch $(C_1-C_4)$Alkyl, $(C_1-C_4)$Alkoxycarbonyl, $(C_1-C_4)$Alkoxy$(C_1-C_4)$alkoxycarbonyl oder $(C_1-C_4)$Alkoxycarbonyl$(C_1-C_4)$alkoxycarbonyl substituiert sein kann;

$R^6$ Wasserstoff oder $(C_1-C_4)$Alkyl,

$R^7$ $(C_1-C_8)$Alkyl; Phenyl oder Benzyl, jeweils bis zu dreifach durch Halogen, $(C_1-C_4)$Alkyl, $(C_1-C_4)$Alkoxy, $CF_3$, $NO_2$ oder $(C_1-C_4)$Alkoxycarbonyl substituiert sein können,

bedeuten, mit der Maßgabe, daß wenn $R^5$ für $CH_3$, X für Sauerstoff und $R^1$ für Wasserstoff steht, $R^2$ und $R^3$ nicht H, Halogen, $(C_1-C_4)$Alkyl, $CF_3$, $(C_1-C_4)$Alkoxy, Phenoxy, Chlorphenoxy, Benzyloxy bedeuten dürfen.

2. Verbindungen der Formel I gemäß Anspruch 1, worin

$R^2$ Fluor, Chlor, Brom, $(C_1-C_4)$Alkoxy, $(C_1-C_4)$Halogenalkoxy, $(C_1-C_4)$Alkylthio, $(C_1-C_4)$Alkylsulfinyl, $(C_1-C_4)$Alkylsulfonyl oder $NO_2$;

$R^3$ Wasserstoff, Chlor, Brom, $(C_1-C_4)$Alkyl, $(C_1-C_4)$Halogenalkyl, $(C_1-C_4)$Alkoxy, $(C_1-C_4)$Halogenalkoxy, $(C_3-C_4)$-Alkinyloxy, $(C_1-C_4)$Alkylthio, $(C_1-C_4)$Alkoxycarbonyl-$(C_1-C_4)$alkylthio oder $(C_1-C_4)$Alkoxycarbonyl$(C_1-C_4)$-alkoxy;

$R^4$ Wasserstoff, $(C_1-C_4)$Alkyl, Benzyl, das ein- bis dreifach durch Halogen oder $(C_1-C_4)$Alkyl substituiert ist, oder $-C(=O)-NHR^7$

$R^5$ $(C_1-C_4)$Alkyl oder Butylen, wobei eine $CH_2$-Gruppe durch Sauerstoff oder Schwefel ersetzt sein kann und das bis zu zweifach durch $(C_1-C_4)$Alkyl substituiert sein kann,

$R^6$ = H und

$R^7$ $(C_1-C_4)$Alkyl

bedeuten.

3. Verbindungen der Formel I gemäß Ansprüchen 1 oder 2, worin $R^1$ = F, $R^2$ = F, Cl, Br, $(C_1-C_4)$Alkoxy, $(C_1-C_4)$Halogenalkoxy, $(C_1-C_4)$Alkylthio oder $(C_1-C_4)$Alkylsulfonyl; $R^3$ = $(C_1-C_4)$Alkoxy, $(C_1-C_4)$Halogenalkoxy, $(C_1-C_4)$Alkylthio, $(C_1-C_4)$Alkoxycarbonyl$(C_1-C_4)$alkoxy, $(C_1-C_4)$Alkoxycarbonyl$(C_1-C_4)$alkylthio oder Propargyloxy, $R^4$ = H, $R^5$ = $(C_1-C_4)$Alkyl, $-\overset{(\alpha)}{C}H_2-CH_2-CH_2-CH_2-$ , $-CH_2-CH_2-O-\overset{(\alpha)}{C}H_2-$ oder $-CH_2-CH_2-S-\overset{(\alpha)}{C}H_2-$ , wobei die letzten drei Reste durch $(C_1-C_4)$Alkyl ein- bis zweifach substituiert sein kann und das C-Atom $(\alpha)$ dieser Reste an C-Atom (4) des Imidazolin(thi)on-Ringes geknüpft ist, und $R^6$ = H bedeuten.

4. Verfahren zur Herstellung von Verbindungen der Formel I gemäß Anspruch 1, 2 oder 3, dadurch gekennzeichnet, daß man eine Verbindung der Formel II

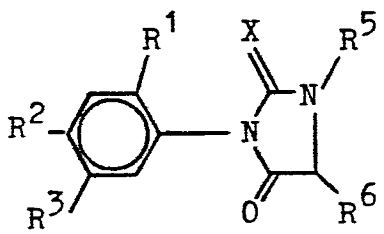

II

reduziert und die erhaltenen Verbindungen gewünschtenfalls verestert.

5. Verwendung von Verbindungen der allgemeinen Formel I von Anspruch 1, 2 oder 3 als Herbizide.

6. Herbizide Mittel, dadurch gekennzeichnet, daß sie eine Verbindung der Formel I von Anspruch 1, 2 oder 3 enthalten.

7. Verfahren zur Bekämpfung von unerwünschten Pflanzen, dadurch gekennzeichnet, daß man eine wirksame Menge einer Verbindung der Formel I von Anspruch 1, 2 oder 3 auf die zu behandelnden Anbauflächen oder die Pflanzen aufbringt.

0234323
HOE 86/F 029

Patentansprüche Österreich:

1. Verfahren zur Herstellung der Verbindungen der allgemeinen Formel I

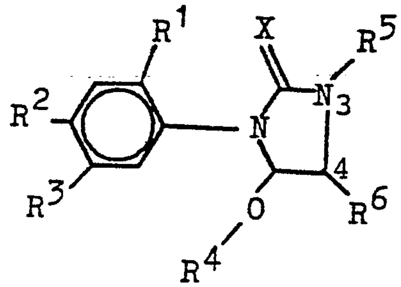

I,

worin

X Sauerstoff oder Schwefel;

$R^1$ Wasserstoff oder Halogen;

$R^2$, $R^3$ unabhängig voneinander Wasserstoff, Halogen, $(C_1-C_4)$Alkyl, $(C_1-C_4)$Halogenalkyl, $(C_1-C_4)$-Alkoxy, $(C_1-C_4)$Halogenalkoxy, $(C_3-C_6)$Cycloalkoxy, $(C_2-C_4)$-Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$-Alkoxy-$(C_1-C_4)$alkoxy; $(C_1-C_4)$Alkoxycarbonyl$(C_1-C_4)$alkoxycarbonyl, $(C_1-C_4)$Halogenalkoxycarbonyl, $(C_1-C_4)$Alkylsulfinyl, $(C_1-C_4)$Alkylsulfonyl, Phenoxy, Phenylthio, Phenylsulfinyl, Phenylsulfonyl wobei der Phenylring jeweils ein- bis dreifach durch Halogen, $(C_1-C_4)$Alkyl, $CF_3$ oder $NO_2$ substituiert sein kann; Pyridyloxy, das ein- oder zweifach durch Halogen oder $CF_3$ substituiert sein kann; Benzyloxy, Phenoxymethyl, die beide im Phenylring ein- bis dreifach durch Halogen, $(C_1-C_4)$Alkyl, $(C_1-C_4)$Alkoxy, $CF_3$ oder $NO_2$ substituiert sein können; $(C_1-C_4)$Alkoxycarbonyl, $(C_1-C_4)$Alkoxycarbonyl$(C_1-C_4)$-alkoxy, $(C_1-C_4)$Alkylthio, $(C_1-C_4)$Alkoxycarbonyl$(C_1-C_4)$-alkylthio, $NO_2$ oder CN,

$R^4$ Wasserstoff, $(C_1-C_4)$Alkyl, Halogen$(C_1-C_4)$alkyl, $(C_3-C_4)$Alkenyl, $(C_3-C_4)$Cycloalkyl, $(C_5-C_6)$Cycloalkenyl, $(C_3-C_4)$Alkinyl, $(C_1-C_4)$Alkoxy-$(C_1-C_4)$alkyl, $(C_1-C_4)$Alkoxycarbonyl$(C_1-C_4)$alkyl, $(C_1-C_4)$Alkylthio$(C_1-C_4)$alkyl; Benzyl, das ein- bis dreifach durch Halogen, $(C_1-C_4)$-

Alkyl, $(C_1-C_4)$Alkoxy, $(C_1-C_4)$Alkoxycarbonyl, $CF_3$, $NO_2$ oder CN substituiert sein kann oder einen Rest der Formel $-\overset{X}{C}-NH-R^7$ ;

$R^5$ Wasserstoff, $(C_1-C_4)$Alkyl, $(C_3-C_4)$Alkenyl, $(C_3-C_4)$-Alkinyl, $(C_3-C_6)$Cycloalkyl, $(C_1-C_4)$Alkoxy$(C_1-C_4)$-alkyl, $(C_1-C_4)$Alkylthio$(C_1-C_4)$alkyl, $(C_1-C_4)$Alkoxy-carbonyl$(C_1-C_4)$alkyl oder eine $(C_4-C_5)$Alkylenkette, die das N-Atom (3) mit dem C-Atom (4) des Imidazolin(thi)on-Ringes verknüpft und bei der eine $CH_2$-Gruppe durch Sauerstoff oder Schwefel substituiert sein kann und die bis zu zweifach durch $(C_1-C_4)$Alkyl, $(C_1-C_4)$Alkoxycarbonyl, $(C_1-C_4)$Alkoxy$(C_1-C_4)$alkoxycarbonyl oder $(C_1-C_4)$Alkoxycarbonyl$(C_1-C_4)$alkoxycarbonyl substituiert sein kann;

$R^6$ Wasserstoff oder $(C_1-C_4)$Alkyl,

$R^7$ $(C_1-C_8)$Alkyl; Phenyl oder Benzyl, jeweils bis zu dreifach durch Halogen, $(C_1-C_4)$Alkyl, $(C_1-C_4)$Alkoxy, $CF_3$, $NO_2$ oder $(C_1-C_4)$Alkoxycarbonyl substituiert sein können, bedeuten, mit der Maßgabe, daß wenn $R^5$ für $CH_3$, X für Sauerstoff und $R^1$ für Wasserstoff steht, $R^2$ und $R^3$ nicht H, Halogen, $(C_1-C_4)$Alkyl, $CF_3$, $(C_1-C_4)$Alkoxy, Phenoxy, Chlorphenoxy, Benzyloxy bedeuten dürfen,

dadurch gekennzeichnet, daß man eine Verbindung der Formel II

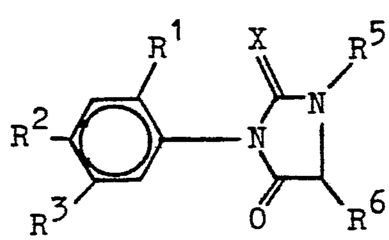

II

reduziert und die erhaltenen Verbindungen gewünschtenfalls verestert.

2. Verfahren gemäß Anspruch 1, wobei in den Formeln I und II $R^2$ = Fluor, Chlor, Brom, $(C_1-C_4)$Alkoxy, $(C_1-C_4)$Halogen-

alkoxy, $(C_1-C_4)$Alkylthio, $(C_1-C_4)$Alkylsulfinyl, $(C_1-C_4)$Alkylsulfonyl oder $NO_2$;

$R^3$ Wasserstoff, Chlor, Brom, $(C_1-C_4)$Alkyl, $(C_1-C_4)$Halogenalkyl, $(C_1-C_4)$Alkoxy, $(C_1-C_4)$Halogenalkoxy, $(C_3-C_4)$-Alkinyloxy, $(C_1-C_4)$Alkylthio, $(C_1-C_4)$Alkoxycarbonyl-$(C_1-C_4)$alkylthio oder $(C_1-C_4)$Alkoxycarbonyl$(C_1-C_4)$-alkoxy;

$R^4$ Wasserstoff, $(C_1-C_4)$Alkyl, Benzyl, das ein- bis dreifach durch Halogen oder $(C_1-C_4)$Alkyl substituiert ist, oder $-\underset{\underset{O}{\|}}{C}-NHR^7$

$R^5$ $(C_1-C_4)$Alkyl oder Butylen, wobei eine $CH_2$-Gruppe durch Sauerstoff oder Schwefel ersetzt sein kann und das bis zu zweifach durch $(C_1-C_4)$Alkyl substituiert sein kann,

$R^6$ = H und

$R^7$ $(C_1-C_4)$Alkyl

bedeuten.

3. Verfahren gemäß Ansprüchen 1 oder 2, wobei in den Formeln I und II $R^1$ = F, $R^2$ = F, Cl, Br, $(C_1-C_4)$Alkoxy, $(C_1-C_4)$Halogenalkoxy, $(C_1-C_4)$Alkylthio oder $(C_1-C_4)$Alkylsulfonyl; $R^3$ = $(C_1-C_4)$Alkoxy, $(C_1-C_4)$Halogenalkoxy, $(C_1-C_4)$Alkylthio, $(C_1-C_4)$Alkoxycarbonyl$(C_1-C_4)$alkoxy, $(C_1-C_4)$Alkoxycarbonyl$(C_1-C_4)$alkylthio oder Propargyloxy, $R^4$ = H, $R^5$ = $(C_1-C_4)$Alkyl, $-CH_2-CH_2-CH_2-CH_2-$ , $-CH_2-CH_2-O-\overset{\alpha}{C}H_2-$ oder $-CH_2-CH_2-S-\underset{\alpha}{C}H_2-$ , wobei die letzten drei Reste durch $(C_1-C_4)$Alkyl ein- bis zweifach substituiert sein kann und das C-Atom α dieser Reste an C-Atom (4) des Imidazolin(thi)on-Ringes geknüpft ist, und $R^6$ = H bedeuten.

4. Verwendung von Verbindungen der allgemeinen Formel I von Anspruch 1, 2 oder 3 als Herbizide.

5. Herbizide Mittel, dadurch gekennzeichnet, daß sie eine Verbindung der Formel I von Anspruch 1, 2 oder 3 enthalten.

6. Verfahren zur Bekämpfung von unerwünschten Pflanzen, dadurch gekennzeichnet, daß man eine wirksame Menge einer Verbindung der Formel I von Anspruch 1, 2 oder 3 auf die zu behandelnden Anbauflächen oder die Pflanzen aufbringt.

Patentansprüche Spanien:

1. Verfahren zur Herstellung der Verbindungen der allgemeinen Formel I

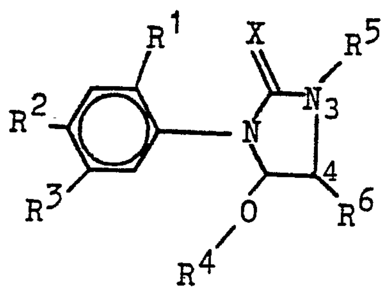

I,

worin

X Sauerstoff oder Schwefel;

$R^1$ Wasserstoff oder Halogen;

$R^2$, $R^3$ unabhängig voneinander Wasserstoff, Halogen, $(C_1-C_4)$Alkyl, $(C_1-C_4)$Halogenalkyl, $(C_1-C_4)$-Alkoxy, $(C_1-C_4)$Halogenalkoxy, $(C_3-C_6)$Cycloalkoxy, $(C_2-C_4)$-Alkenyloxy, $(C_2-C_4)$Halogenalkenyloxy, $(C_5-C_6)$Cycloalkenyloxy, $(C_3-C_4)$Alkinyloxy, $(C_3-C_4)$Halogenalkinyloxy, $(C_1-C_4)$-Alkoxy-$(C_1-C_4)$alkoxy; $(C_1-C_4)$Alkoxycarbonyl$(C_1-C_4)$alkoxycarbonyl, $(C_1-C_4)$Halogenalkoxycarbonyl, $(C_1-C_4)$Alkylsulfinyl, $(C_1-C_4)$Alkylsulfonyl, Phenoxy, Phenylthio, Phenylsulfinyl, Phenylsulfonyl wobei der Phenylring jeweils ein- bis dreifach durch Halogen, $(C_1-C_4)$Alkyl, $CF_3$ oder $NO_2$ substituiert sein kann; Pyridyloxy, das ein- oder zweifach durch Halogen oder $CF_3$ substituiert sein kann; Benzyloxy, Phenoxymethyl, die beide im Phenylring ein- bis dreifach durch Halogen, $(C_1-C_4)$Alkyl, $(C_1-C_4)$Alkoxy, $CF_3$ oder $NO_2$ substituiert sein können; $(C_1-C_4)$Alkoxycarbonyl, $(C_1-C_4)$Alkoxycarbonyl$(C_1-C_4)$-alkoxy, $(C_1-C_4)$Alkylthio, $(C_1-C_4)$Alkoxycarbonyl$(C_1-C_4)$-alkylthio, $NO_2$ oder CN,

$R^4$ Wasserstoff, $(C_1-C_4)$Alkyl, Halogen$(C_1-C_4)$alkyl, $(C_3-C_4)$Alkenyl, $(C_3-C_4)$Cycloalkyl, $(C_5-C_6)$Cycloalkenyl, $(C_3-C_4)$Alkinyl, $(C_1-C_4)$Alkoxy-$(C_1-C_4)$alkyl, $(C_1-C_4)$Alkoxycarbonyl$(C_1-C_4)$alkyl, $(C_1-C_4)$Alkylthio$(C_1-C_4)$alkyl; Benzyl, das ein- bis dreifach durch Halogen, $(C_1-C_4)$-

Alkyl, $(C_1-C_4)$Alkoxy, $(C_1-C_4)$Alkoxycarbonyl, $CF_3$, $NO_2$ oder CN substituiert sein kann oder einen Rest der Formel $-\underset{\underset{X}{\|}}{C}-NH-R^7$ ;

$R^5$ Wasserstoff, $(C_1-C_4)$Alkyl, $(C_3-C_4)$Alkenyl, $(C_3-C_4)$-Alkinyl, $(C_3-C_6)$Cycloalkyl, $(C_1-C_4)$Alkoxy$(C_1-C_4)$-alkyl, $(C_1-C_4)$Alkylthio$(C_1-C_4)$alkyl, $(C_1-C_4)$Alkoxy-carbonyl$(C_1-C_4)$alkyl oder eine $(C_4-C_5)$Alkylenkette, die das N-Atom (3) mit dem C-Atom (4) des Imidazolin(thi)on-Ringes verknüpft und bei der eine $CH_2$-Gruppe durch Sauerstoff oder Schwefel substituiert sein kann und die bis zu zweifach durch $(C_1-C_4)$Alkyl, $(C_1-C_4)$Alkoxycarbonyl, $(C_1-C_4)$Alkoxy$(C_1-C_4)$alkoxycarbonyl oder $(C_1-C_4)$Alkoxycarbonyl$(C_1-C_4)$alkoxycarbonyl substituiert sein kann;

$R^6$ Wasserstoff oder $(C_1-C_4)$Alkyl,

$R^7$ $(C_1-C_8)$Alkyl; Phenyl oder Benzyl, jeweils bis zu dreifach durch Halogen, $(C_1-C_4)$Alkyl, $(C_1-C_4)$Alkoxy, $CF_3$, $NO_2$ oder $(C_1-C_4)$Alkoxycarbonyl substituiert sein können, bedeuten, mit der Maßgabe, daß wenn $R^5$ für $CH_3$, X für Sauerstoff und $R^1$ für Wasserstoff steht, $R^2$ und $R^3$ nicht H, Halogen, $(C_1-C_4)$Alkyl, $CF_3$, $(C_1-C_4)$Alkoxy, Phenoxy, Chlorphenoxy, Benzyloxy bedeuten dürfen,

dadurch gekennzeichnet, daß man eine Verbindung der Formel II

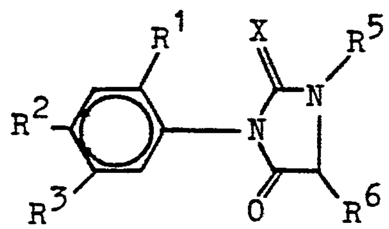

II

reduziert und die erhaltenen Verbindungen gewünschtenfalls verestert.

2. Verfahren gemäß Anspruch 1, wobei in den Formeln I und II $R^2$ = Fluor, Chlor, Brom, $(C_1-C_4)$Alkoxy, $(C_1-C_4)$Halogen-

alkoxy, $(C_1-C_4)$Alkylthio, $(C_1-C_4)$Alkylsulfinyl, $(C_1-C_4)$Alkylsulfonyl oder $NO_2$;

$R^3$ Wasserstoff, Chlor, Brom, $(C_1-C_4)$Alkyl, $(C_1-C_4)$Halogenalkyl, $(C_1-C_4)$Alkoxy, $(C_1-C_4)$Halogenalkoxy, $(C_3-C_4)$-Alkinyloxy, $(C_1-C_4)$Alkylthio, $(C_1-C_4)$Alkoxycarbonyl-$(C_1-C_4)$alkylthio oder $(C_1-C_4)$Alkoxycarbonyl$(C_1-C_4)$-alkoxy;

$R^4$ Wasserstoff, $(C_1-C_4)$Alkyl, Benzyl, das ein- bis dreifach durch Halogen oder $(C_1-C_4)$Alkyl substituiert ist, oder $-\underset{\underset{O}{\|}}{C}-NHR^7$,

$R^5$ $(C_1-C_4)$Alkyl oder Butylen, wobei eine $CH_2$-Gruppe durch Sauerstoff oder Schwefel ersetzt sein kann und das bis zu zweifach durch $(C_1-C_4)$Alkyl substituiert sein kann,

$R^6$ = H und

$R^7$ $(C_1-C_4)$Alkyl

bedeuten.

3. Verfahren gemäß Ansprüchen 1 oder 2, wobei in den Formeln I und II $R^1$ = F, $R^2$ = F, Cl, Br, $(C_1-C_4)$Alkoxy, $(C_1-C_4)$Halogenalkoxy, $(C_1-C_4)$Alkylthio oder $(C_1-C_4)$Alkylsulfonyl; $R^3$ = $(C_1-C_4)$Alkoxy, $(C_1-C_4)$Halogenalkoxy, $(C_1-C_4)$Alkylthio, $(C_1-C_4)$Alkoxycarbonyl$(C_1-C_4)$alkoxy, $(C_1-C_4)$Alkoxycarbonyl$(C_1-C_4)$alkylthio oder Propargyloxy, $R^4$ = H, $R^5$ = $(C_1-C_4)$Alkyl, $-CH_2-CH_2-CH_2-CH_2-$ , $-CH_2-CH_2-O-\overset{\alpha}{C}H_2-$ oder $-CH_2-CH_2-S-\underset{\alpha}{C}H_2-$ , wobei die letzten drei Reste durch $(C_1-C_4)$Alkyl ein- bis zweifach substituiert sein kann und das C-Atom α dieser Reste an C-Atom (4) des Imidazolin(thi)on-Ringes geknüpft ist, und $R^6$ = H bedeuten.

Europäisches Patentamt

**EUROPÄISCHER RECHERCHENBERICHT**

Nummer der Anmeldung 0234323

EP 87 10 1242

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | WO-A-8 401 383 (OTSUKA)<br>* Spalten 1-3,6,7 * & US-A-4 578 107 (Kat. P,X)<br>--- | 1-7 | C 07 D 233/40<br>C 07 D 233/42<br>C 07 D 471/04<br>C 07 D 513/04<br>C 07 D 498/04<br>A 01 N 43/50<br>A 01 N 43/90 |
| A | US-A-4 179 276 (CHENG)<br>--- | | |
| A | EP-A-0 070 389 (SUMITOMO)<br>--- | | |
| A | DE-A-2 658 058 (BAYER)<br>----- | | |
| | | | RECHERCHIERTE SACHGEBIETE (Int. Cl.4) |
| | | | C 07 D 233/00<br>C 07 D 471/00<br>C 07 D 513/00<br>C 07 D 498/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 16-05-1987 | DE BUYSER I.A.F. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument